# FASCICULE DE BREVET EUROPEEN

(11) **EP 3 907 060 B1**
(45) Date de publication et mention de la délivrance du brevet: **08.05.2024**
(21) Numéro de dépôt: 21172382.0
(22) Date de dépôt: 06.05.2021
(51) Int. Cl.: B29C 65/20, A61M 39/14, B29K 101/12

(54) **LAME CHAUFFANTE MULTI-USAGE POUR CONNEXION DE TUBES POUVANT ÊTRE PLEINS, ET MACHINE EQUIPÉE D'UNE TELLE LAME**
MEHRFACH VERWENDBARE HEIZLEISTE FÜR DIE VERBINDUNG VON ROHREN, DIE GEFÜLLT SEIN KÖNNEN, UND MASCHINE MIT EINER SOLCHEN LEISTE
MULTI-USE HEATING BLADE FOR CONNECTING TUBES THAT MAY BE FULL, AND MACHINE PROVIDED WITH SUCH A BLADE

(30) Priorité: 07.05.2020 FR 2004568
(43) Date de publication de la demande: 10.11.2021
(73) Titulaire: MGA Technologies, 69380 Civrieux d'Azergues (FR)
(72) Inventeur: de MALLIARD, Hervé, 69160 TASSIN LA DEMI-LUNE (FR)
(74) Mandataire: Cabinet Laurent & Charras

(56) Documents cités:
- JP-A- H09 206 383
- US-A- 4 864 101
- US-B2- 7 122 094

## Description

La présente invention a trait à une lame chauffante multi-usage, adaptée notamment à des opérations de connexion par thermo-soudure de tubes souples pouvant être pleins. L'invention concerne également une machine de thermo-soudure utilisant une telle lame chauffante.

Dans les domaines biomédical et pharmaceutique, la thermo-soudure de pièces plastiques est fréquemment employée pour la soudure de pièces thermoplastiques, par exemple des tubes souples en matière thermoplastique.

Par « tubes souples en matière thermoplastique », l'on entend des tubes réalisés en un matériau polymère présentant des propriétés élastiques, tel que l'élastomère.

L'invention vise en particulier la connexion de tels tubes, particulièrement pour des tubes ayant un diamètre extérieur allant de 5 à 30 millimètres.

L'invention se rapporte à des opérations de connexion de tubes souples dans le domaine biomédical et pharmaceutique.

De telles opérations de connexion de tubes souples consistent typiquement à assembler par thermo-soudure deux tubes en matière thermoplastique bout à bout.

La connexion entre les tubes est réalisée en sectionnant des tubes à haute température, formant deux extrémités, qui sont fondues puis mises en contact entre elles. De telles opérations de connexion sont habituellement réalisées grâce à une lame chauffante, intégrée dans une machine de connexion.

Les lames chauffantes permettant de réaliser des opérations de connexion de tubes par thermo-soudure se divisent en deux catégories : les lames chauffantes mono-usage et les lames chauffantes multi-usages. Les documents US 7122094, JPH09 206383 et US 4864101 décrivent de telles lames chauffantes.

Les lames chauffantes mono-usage sont généralement constituées d'une simple plaque métallique à base de nickel, pouvant être enrichie par exemple avec du chrome, de sorte à accroître la résistivité de l'alliage, et par conséquence la résistance ohmique de la plaque. La circulation d'un courant au sein de la plaque permet un dégagement de chaleur par effet Joule. Une telle plaque bien que simple d'obtention, est à utilisation unique. Ainsi, après réalisation d'une opération de connexion de tubes, la lame chauffante doit être remplacée par une lame neuve, ou bien doit être nettoyée et décontaminée.

Les lames chauffantes multi-usages relèvent en général d'une constitution plus complexe que les lames mono-usage. Les lames chauffantes multi-usages comprennent en effet souvent une pièce active en contact avec le tube, et un dispositif de chauffe. La pièce active comprend généralement une partie tranchante destinée à sectionner le tube, et une partie de soudure destinée à permettre la mise en température des sections de tube, préalablement sectionnés pour la soudure. La partie active est portée à température par l'intermédiaire de la chaleur dégagée par le dispositif de chauffe.

Les tubes à connecter entre eux sont souvent utilisés à l'état neuf après avoir été décontaminés avant qu'il ne soit réalisé une opération de connexion de tubes.

Cependant, dans certaines situations, il est nécessaire de réaliser des connexions entre deux tubes remplis de matières, dits tubes pleins. Par exemple, afin de réaliser des transferts de cultures biologiques ou de composés organiques entre deux contenants, il est nécessaire de réaliser une connexion stérile entre un premier tube relié au premier contenant et un deuxième tube relié au deuxième contenant, ceci afin de connecter entre eux les deux contenants.

L'invention vise en particulier des opérations de connexion de tubes pleins, notamment la connexion entre eux de l'un au moins des tubes pleins.

L'opération de connexion de deux tubes pleins se déroule en général comme suit. Dans une étape de compression, la machine de soudage compresse et déforme les deux tubes jusqu'à obturation complète des tubes. Puis, les deux tubes sont sectionnés dans une étape de sectionnement, et après retournement, connectés par thermo-soudure lors d'une étape de soudure.

L'étape de compression relève d'une grande importance. En effet, outre le fait que l'obturation des tubes sectionnés permet d'éviter de contaminer les composés liquides éventuellement présents dans les récipients, en les mettant au contact de l'air ambiant extérieur, une telle étape évite le déversement du liquide lui-même contaminant en dehors des tubes.

Une fuite de liquide en dehors des tubes est en effet particulièrement délétère dans l'opération de connexion de tubes pleins, car des matières contenues peuvent alors entrer en contact avec la lame chauffante et se mélanger avec le thermoplastique en fusion, ce qui nuit à une bonne réalisation de l'étape de soudure.

En présence d'une quantité trop importante de liquide, la soudure est même impossible à réaliser.

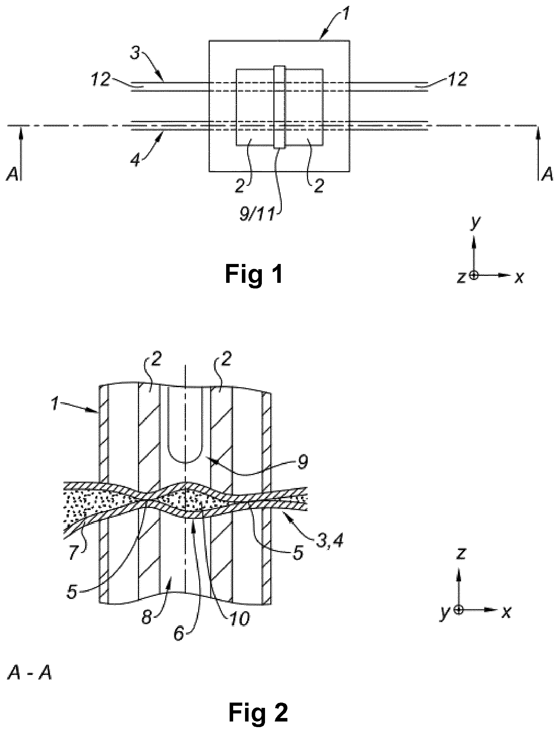

Généralement, comme illustré sur les figures 1 et 2, la machine **1** est équipée de moyens de compression, tels que deux paires d'enclumes **2** placés de part et d'autre du premier tube **3** et du deuxième tube **4.** Lors de l'étape de compression, représentée plus particulièrement sur la vue en coupe de la figure 2, les enclumes **2** se déplacent perpendiculairement à la direction d'extension des tubes **3, 4,** de sorte à venir presser chacun des tubes **3, 4** en deux points **5** de compression, disposés de part et d'autre d'une partie **6** centrale, ce qui a pour effet de plaquer entre elles les parois **7** de tubes, permettant ainsi une obturation hermétique des tubes **3, 4.**

Puis, les enclumes **2** s'écartent très légèrement dans la direction d'extension des tubes, ce qui tend le premier tube **3,** écrase la partie **6** centrale, et permet de laisser un interstice **8** au niveau de la partie **6** centrale pour qu'une lame **9** puisse venir réaliser les étapes de sectionnement et de soudure.

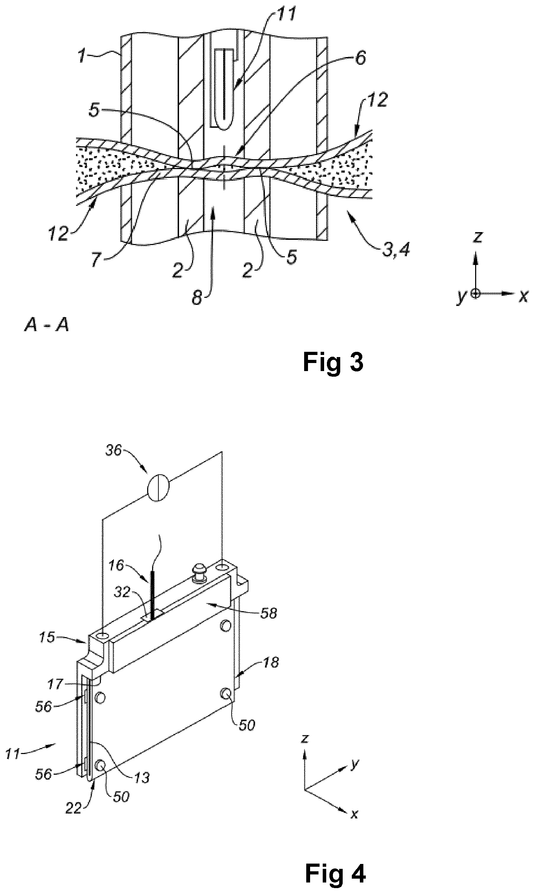

Un dispositif de retournement (non représenté) permet de retourner les tubes **3, 4,** ce qui permet de mettre face à face le premier tube **3** et le deuxième tube **4,** la lame étant interposée entre les tubes **3, 4** de manière à maintenir les extrémités dans un état de fusion (voir figure 3). La lame est ensuite retirée, les extrémités du premier tube **3** et du deuxième tube **4** sont pressées l'une contre l'autre de façon à permettre la réalisation de la soudure.

Une telle étape de compression permet certes d'obturer hermétiquement le tube **3, 4** en deux points, empêchant le déversement du contenu complet du tube **3, 4,** et la pollution du contenu du tube **3, 4.** Cependant, la compression n'empêche pas la formation d'une poche **10** de liquide résiduel, puisque le liquide présent dans la partie **6** centrale n'est pas évacué au moment de la compression. Dans une telle situation, la réalisation d'une soudure est impossible du fait d'une quantité trop importante de liquide.

Afin d'éliminer la poche **10** de liquide résiduel après compression, il a été recherché de limiter au plus possible la dimension de la partie centrale, mais un tel choix technique est limitant quant au type de lame chauffante à utiliser. En effet, seules les lames mono-usage, qui présentent une épaisseur suffisamment fine, peuvent s'introduire dans l'interstice **8.**

Il a certes été essayé de réduire l'épaisseur des lames multi-usages, mais de nombreux problèmes ont été rencontrés du fait de l'affinage des composants de la lame multi-usages. Ainsi, il a été constaté la difficulté de contrôler efficacement la température en surface de la lame, ainsi que l'insuffisance de la résistance mécanique de la lame pour faire face aux efforts engendrés par le sectionnement du tube, en particulier pour les tubes présentant un diamètre important, tout en conservant une épaisseur réduite de ladite lame.

De manière alternative, il a été également proposé, comme décrit par exemple dans les documents EP 1555111 ou DE 2401800, des mécanismes d'expulsion de liquide. De tels mécanismes viennent presser la partie centrale du tube, et tout en maintenant l'effort de pression sur le tube, s'éloignent de la partie centrale. De cette façon, le liquide est évacué de la partie centrale du tube, et un interstice **8** de dimension suffisante pour autoriser un passage d'une lame multi-usage pour effectuer l'étape de sectionnement et de soudure.

Cependant, de tels mécanismes sont complexes à mettre en oeuvre, couteux, et encombrants.

Par ailleurs, les lames multi-usages connues, suite à la réalisation d'opérations de connexion de tubes pleins ressortent souillées et contaminées par le contenu des tubes à connecter entre eux, et souvent localement recouvertes de résidus de thermoplastique issus des tubes. De telles lames multi-usages ne peuvent pas être immédiatement réutilisées pour effectuer une autre opération de connexion de tubes pleins, mais nécessitent un nettoyage ainsi qu'une décontamination avant d'être réutilisées.

De telles opérations nécessitent d'attendre le refroidissement de la lame multi-usage, pour permettre son démontage et la réalisation du nettoyage et de la décontamination, puis un remontage au sein de la machine, ce qui est long et fastidieux, en particulier lorsqu'un nombre relativement important de tubes sont à connecter entre eux, par exemple dans le cas des machines de production.

L'invention vise à résoudre les inconvénients précités.

Un premier objet est de proposer une lame chauffante multi-usage pouvant être réutilisée immédiatement après la réalisation d'une opération de connexion entre deux tubes même pleins.

Un deuxième objet est de proposer une lame chauffante multi-usage permettant de rendre possible une opération de connexion de tubes plein par procédé de thermo-soudure, sans faire usage de mécanismes d'expulsion additionnels.

Un troisième objet est de proposer une lame chauffante telle que présentée ci-dessus apte à pouvoir être régulée efficacement en température, tout en présentant une résistance mécanique suffisante nonobstant une épaisseur réduite et un encombrement minimal, et conservant au maximum son intégrité après plusieurs opérations de connexion.

Un quatrième objet est de proposer une lame chauffante multi-usage fiable et sûre d'utilisation.

Un cinquième objet est de proposer une machine de chauffe comprenant une lame chauffante multi-usage telle que présentée ci-dessus.

Il est proposé en premier lieu une lame chauffante multi-usage apte à réaliser plusieurs opérations de thermo-soudure d'un tube plein, comprenant une feuille métallique, et un dispositif de chauffe résistif apte à augmenter la température de la feuille à une température prédéterminée, la feuille comprenant une face externe destinée à entrer en contact avec un tube, la face externe étant munie d'un revêtement anti-adhérent.

Une telle lame chauffante multi-usage peut réaliser des opérations de connexion de tubes pleins, notamment grâce à son épaisseur réduite, lui permettant de s'introduire dans un interstice limité. De plus, la lame chauffante multi-usage dispose d'une bonne tenue, notamment mécanique, au fur et à mesure des utilisations, et permet la réalisation d'une soudure de qualité.

Par ailleurs, le dispositif de chauffe se présente sous la forme d'une plaque, la feuille métallique étant pliée en deux de sorte à définir un espace interne entre deux faces internes, espace interne dans lequel est disposé le dispositif de chauffe, ce dernier étant pris en sandwich entre les deux faces internes et solidarisé à ces dernières par collage au moyen d'un adhésif constitué d'une colle époxy mono-composant.

Les caractéristiques suivantes peuvent être prévues, seules ou en combinaison :
▪ le revêtement présente plusieurs couches superposées les unes sur les autres ;
▪ le revêtement comprend du PTFE ;
▪ la lame comprend un support solidaire de la feuille et du dispositif de chauffe, le support comprenant un corps de liaison, un premier bras et un deuxième bras s'étendant dans le même sens de sorte à former un U, les bras intégrant une surface d'appui, la lame étant munie de moyens de fixation destinée à plaquer la feuille contre la surface d'appui ;
▪ les bras comprennent une surface de base, les bras étant munis de bossages faisant saillie de la surface de base, définissant chacun un plan d'appui décalé de la surface de base, les plans d'appui étant coplanaires et formant ensemble la surface d'appui ;
▪ le dispositif de chauffe est muni d'un circuit électrique comprenant une piste métallique, le circuit étant disposé sur un substrat ;
▪ le substrat est réalisé en un matériau appartenant à la famille des polymères polyimides;
▪ le circuit s'étend en formant un motif présentant un contour fictif fermé comprenant des portions latérales, une portion de contact, toutes les deux sensiblement rectilignes, ainsi que d'une portion arquée décrivant un arc-de-cercle, les portions latérales étant chacune reliées d'une part à la portion arquée, et d'autre part à la portion de contact ;
▪ la feuille est pliée en définissant une pliure, le circuit comprend une zone de surchauffe au voisinage de la pliure, et une zone de chauffe à distance de la pliure, la fraction de piste s'étendant au sein de la zone de surchauffe présentant une résistivité surfacique supérieure à la fraction de piste s'étendant au sein de la zone de chauffe ;
▪ la lame comprend un dispositif de mesure de température disposé dans une entaille pratiquée au sein du dispositif de chauffe, le dispositif de mesure de température présentant une épaisseur inférieure à l'épaisseur du dispositif de chauffe ;
▪ la feuille présente une épaisseur de 0.15 à 0.25 millimètre, et le dispositif de chauffe présente une épaisseur de 0.18 à 0.22 millimètre, la lame chauffante présentant une épaisseur de 0.7 à 0.8 millimètre.

Il est proposé en deuxième lieu une machine comprenant une lame chauffante tel que présentée ci-dessus.

D'autres caractéristiques et avantages de l'invention apparaîtront plus clairement et de manière concrète à la lecture de la description ci-après de modes de réalisation, laquelle est faite en référence aux dessins annexés dans lesquels :
La figure 1 représente une vue schématique de dessus d'un exemple de machine de thermo-soudure ;
La figure 2 représente une vue schématique en coupe selon le plan de coupe A-A de la figure 1 de la machine de thermo-soudure, la machine de thermo-soudure étant munie d'une lame selon l'art antérieur, la machine étant représentée en cours de réalisation d'une opération de thermo-soudure ;
La figure 3 représente une vue schématique selon le plan de coupe A-A de la figure 1, la machine de thermo-soudure étant munie d'une lame chauffante multi-usage, la machine étant représentée en cours de réalisation d'une opération de thermo-soudure ;
La figure 4 représente une vue schématique en perspective d'une lame chauffante multi-usage ;
La figure 5 représente une vue schématique de face de la lame chauffante multi-usage ;
La figure 6 représente une vue schématique latérale de la lame, ainsi qu'un médaillon d'un détail sur une partie basse de la lame ;
La figure 7 représente une vue schématique en perspective du dispositif de chauffe ;
La figure 8 représente une vue schématique en perspective d'un support de la lame chauffante multi-usage.

L'on se réfère aux figures 1 et 3 qui représentent un exemple d'une machine **1** munie d'une lame chauffante **11** multi-usage, c'est-à-dire une lame chauffante destinée à être utilisée au cours de plusieurs opérations de thermo-soudures successives.

Dans la suite de la description, l'expression « lame **11** » désigne la lame chauffante **11** multi-usage.

Comme représenté sur la figure 3, pour réaliser l'opération de connexion entre les tubes **3, 4,** des enclumes **2** disposées de part et d'autre de la partie **6** centrale sont déplacées, venant comprimer les parois **7** du tube **3, 4.** Puis, la lame **11,** préalablement portée à une température permettant la fusion du thermoplastique constituant le tube **3, 4** (typiquement entre 180°C et 210°C), se déplace par exemple en translation. Les tubes **3, 4** sont sectionnés en deux sections **12** de tubes disposés l'un à côté de l'autre, formant respectivement par exemple quatre sections **12** de tube. Puis après avoir été pivotées, deux sections **12** de tubes sont pressées contre la lame **11,** encore à température de fusion du thermoplastique formant le tube **3, 4.** La lame **11** est ensuite retirée et les sections **12** de tube sont pressées entre elles jusqu'à solidification des zones en fusion. Les enclumes **2** sont ensuite retirées.

La machine 1 ainsi que la lame **11** trouvent une application particulièrement avantageuse pour des opérations de connexion pour des tubes **3, 4** présentant des diamètres allant par exemple de 5 à 30 millimètres.

La machine **1** ainsi que la lame **11,** trouvent des utilisations avantageuses dans les domaines de l'industrie pharmaceutique, alimentaire, médicale, biotechnologique et chimique.

L'on définit par rapport à un tube **3, 4** un repère XYZ formant un trièdre, comprenant trois axes perpendiculaires deux à deux, à savoir :
- un axe X qui définit une direction longitudinale, horizontale, confondue avec la direction d'extension du tube **3, 4** ;
- un axe Y définissant une direction transversale, horizontale, qui avec l'axe X définit un plan XY horizontal ;
- un axe Z, définissant une direction verticale, perpendiculaire au plan XY horizontal.

Telle que représentée sur les figures 4 à 6, la lame **11** inclut une pièce active, telle qu'une feuille **13** réalisée en un matériau métallique, et un dispositif **14** de chauffe. La feuille **13** et le dispositif **14** de chauffe sont en contacts l'un de l'autre, par exemple à l'aide d'un support **15.**

Le dispositif **14** de chauffe est une source de chaleur, avantageusement contrôlée par l'intermédiaire d'un dispositif de mesure de température, tel qu'un capteur **16** de température, tandis que la feuille métallique **13** est destinée à entrer en contact avec le tube **3, 4** pour réaliser le sectionnement et la soudure. De cette manière, préalablement à l'étape de sectionnement et de soudure, la lame **11** peut être portée à une température prédéterminée, par l'intermédiaire du dispositif **14** de chauffe.

La feuille métallique **13** présente une épaisseur se situant avantageusement dans une plage allant de 0.15 à 0.25 millimètre. Il a été constaté qu'une telle épaisseur est compatible avec la réalisation de connexion de tubes pleins, puisqu'elle permet de laisser un interstice **8** suffisamment faible entre les enclumes **2** pour ne laisser qu'une quantité négligeable de liquide dans la partie **6** centrale du tube **3, 4.**

Avantageusement, la feuille métallique **13** présente une forme rectangulaire, en définissant un premier côté **17,** s'étendant transversalement, et un deuxième côté **18** vertical. De cette manière, la lame **11** peut se positionner facilement sur un support **15,** en occupant un espace minimal.

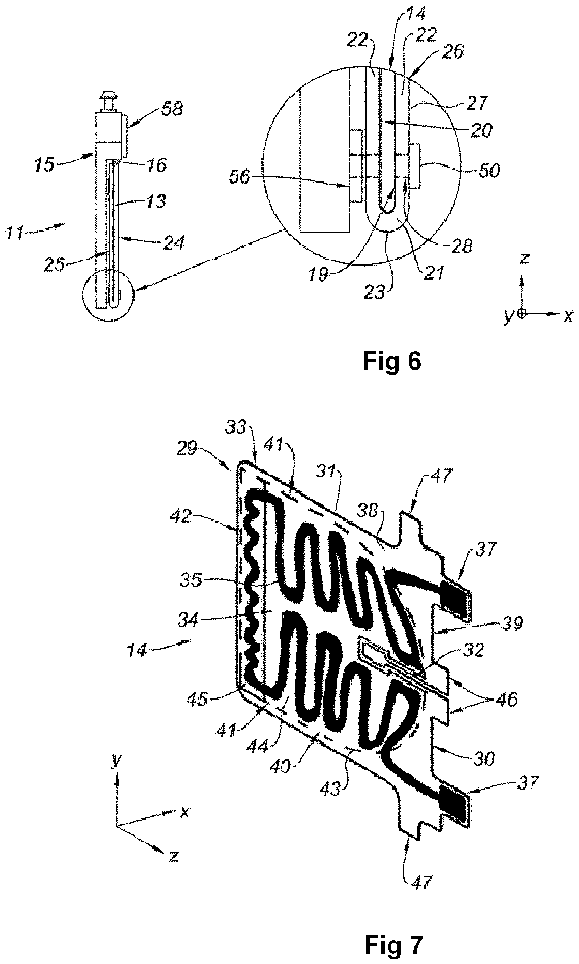

Comme représentés sur les figures 4 et 6, et en particulier sur la figure 6, la feuille métallique **13** est pliée en deux de sorte à définir un espace interne entre une première face interne **19** et une deuxième face interne **20,** l'espace interne permettant de recevoir le dispositif **14** de chauffe. Le dispositif **14** de chauffe est ainsi pris en sandwich, en étau entre les deux faces **19, 20** internes.

Une telle disposition permet de réduire l'épaisseur totale de la lame 11.

La lame **11** peut alors être introduite dans un interstice **8** de dimension minimale, et rendre ainsi réalisable une opération de connexion de tubes **3, 4** pleins sans nécessiter un mécanisme d'expulsion de liquide.

La feuille **13** comprend une pliure **21** transversale, formée par repliement de la feuille **13** sur elle-même. Avantageusement, la feuille **13** est pliée en formant deux ailes **22** symétriques l'une de l'autre par rapport à la pliure.

La feuille **13** comprend une partie **23** tranchante, transversale, passant par exemple confondue par le milieu de la pliure **21.** La pliure **21,** du fait de sa finesse, permet de rendre la partie **23** tranchante suffisamment coupante de sorte à permettre le sectionnement d'un tube thermoplastique porté à température de fusion.

La feuille **13** pliée entoure au moins partiellement le dispositif **14** de chauffe. De cette manière, le dispositif **14** de chauffe est mis en position au sein de la feuille **13.** En outre, la pliure **21** évite l'entrée de matière entre les faces **19, 20** internes, évitant ainsi le contact direct entre du thermoplastique fondu, et/ou du liquide avec le dispositif **14** de chauffe lors de l'étape de sectionnement.

De manière à favoriser les échanges thermiques par conduction, les faces internes **19, 20** sont toutes les deux en contact du dispositif **14** de chauffe. Ce dernier est même solidarisé auxdites faces internes **19, 20** par collage. La colle mise en oeuvre est une colle époxy mono composant spécifique, polymérisant à chaud. Typiquement, un tel adhésif est commercialisé sous la référence LOCTITE^{®} EU 9514 par HENKEL. Cet adhésif présente la caractéristique de posséder une résistance élevée au pelage et au cisaillement. De manière encore plus spécifique, si cet adhésif voit sa tenue à la contrainte diminuer avec la température, elle demeure cependant suffisante dans la plage de température du fonctionnement de la lame **11,** c'est-à-dire entre 170 et 230 °C. Ce faisant, de par la mise en oeuvre d'un tel adhésif, on n'observe pas de décollement de la feuilles métallique **13** du dispositif de chauffe **14,** même ces températures, garantissant la pérennité de la lame **11,** et son caractère multi-usage sans affecter sa résistance mécanique et sa tenue dans le temps.

Comme déjà indiqué, la feuille **13** est réalisée en un matériau métallique, par exemple l'acier. Un tel matériau peut supporter d'être porté à des températures permettant de réaliser la soudure des tubes, par exemple autour de 200°C à 230 °C, sans subir de modification de caractéristiques mécaniques.

La feuille **13** présente une première face **24** externe, opposée à la première face **19** interne, et une deuxième face **25** externe, opposée à la deuxième face **20** interne, destinées à entrer toutes les deux en contact du tube **3, 4,** lors de l'étape de sectionnement et de soudure.

De manière à éviter l'adhérence de l'extrémité du tube en fusion et des résidus de matières éventuellement présentes dans les tubes, la feuille **13** comprend un revêtement **26** anti-adhérent, préférentiellement disposé sur la première face **24** externe, et/ou la deuxième face **25** externe.

Le revêtement **26** empêche l'adhérence du thermoplastique en fusion sur une face **24, 25** externe, lorsque la lame **11** est retirée après la réalisation de l'étape de soudure. De cette manière, la réutilisation de la lame **11** pour plusieurs opérations de connexion de tubes **3, 4** est possible sans qu'il soit nécessaire de procédure à un nettoyage ou une décontamination.

Un tel revêtement **26,** bien qu'ayant une épaisseur négligeable par rapport à l'épaisseur de la lame, est volontairement représenté agrandi sur la figure 6. Avantageusement, le revêtement comprend des matériaux reconnus anti-adhérents comme des PTFE (polytétrafluoroéthylène), PFA (perfluoroalkoxy) ou FEP (Fluorinated Ethylene Propylene) combinés entre eux, ou non. De tels matériaux offrent un compromis optimal entre leur pouvoir antiadhésif et leur point de fusion bien supérieur à la température de chauffe de la lame **11.**

Dans des modes de réalisation avantageux, le revêtement **26** comprend plusieurs couches **27** superposées, afin d'être suffisamment durables pour effectuer une pluralité d'opérations. De manière à permettre une homogénéité efficace de la température des faces **24, 25** externes, il est préférable de ne prévoir qu'un minium de couches (typiquement deux couches) **27** superposées.

Comme représenté en pointillé sur la figure 6, la feuille métallique **13** présente avantageusement une pluralité de d'orifices **28** de sorte à permettre de réaliser sa fixation sur un support **15.**

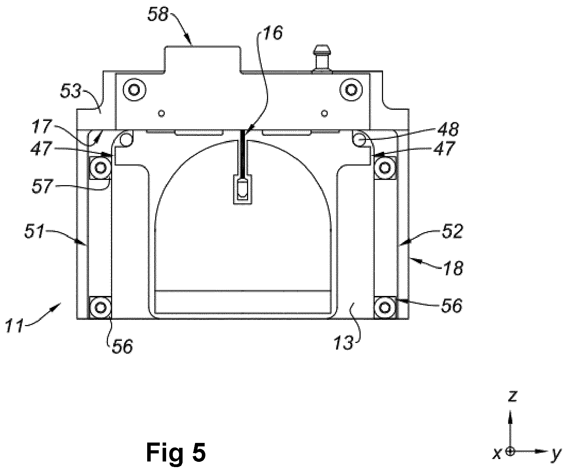

L'on décrit à présent le dispositif **14** de chauffe en se référant aux figures 5 et 7.

Le dispositif **14** de chauffe se présente par exemple sous la forme d'une plaque **29,** de sorte à pouvoir être prise en sandwich entre les faces internes **19, 20** de la feuille **13.** Le dispositif **14** de chauffe offre ainsi une épaisseur minimale, avantageusement entre 0.18 millimètre et 0.22 millimètre.

Par ailleurs, la forme de plaque permet au dispositif **14** de chauffe d'avoir un contact plan sur plan contre les faces internes **19, 20** de la feuille métallique **13,** facilitant la mise en position et le maintien en position du dispositif de chauffe **14** entre les faces internes **19, 20.** En outre, les échanges thermiques par conduction entre le dispositif **14** de chauffe et les faces internes **19, 20** de la feuille métallique **13** sont favorisés, et en outre optimisés par l' adhésif précité, ce qui facilite le contrôle de température de la lame **11.**

La plaque **29** présente par exemple une forme sensiblement rectangulaire, définissant un pourtour intégrant une première rive **30,** transversale et une deuxième rive **31** verticale.

Dans le mode de réalisation représenté, le dispositif **14** de chauffe comprend une entaille **32,** s'étendant par exemple à partir d'une première rive **30** dans le centre du dispositif **14** de chauffe. L'entaille **32** définit un emplacement pour permettre l'insertion d'un capteur **16** de température, ce qui limite l'encombrement de la lame **11,** et offre la possibilité d'effectuer une mesure de la température au coeur, c'est-à-dire au centre du dispositif **14** de chauffe. De cette façon, il est possible d'obtenir un relevé de température non perturbé par des flux d'air convectifs. La régulation de la température de la lame **11** est ainsi facilitée.

Le dispositif **14** de chauffe comprend avantageusement un substrat **33** comprenant un circuit électrique **34,** formé par exemple d'une piste métallique **35** reliée à un générateur externe **36** par l'intermédiaire de cosses **37.** Le circuit électrique **34** est par exemple disposé sur le substrat **33.** De cette manière, la circulation d'un courant introduit par le générateur externe **36** engendre un dégagement de chaleur par effet Joule, qui permet de porter la lame **11** à température. Ainsi, une telle caractéristique permet de rendre résistif le dispositif **14** de chauffe.

Avantageusement, le substrat **33** comprend une paroi chauffante **38** munie du circuit électrique **34,** et une paroi de fixation **39** destinée à être solidarisée à une face **17** interne, également dénommé premier côté.

Dans le mode de réalisation représenté, la paroi de fixation **39** est solidarisée par l'intermédiaire d'un adhésif (non visible sur les figures), par exemple une colle époxy mono-composant, qui présente l'avantage de polymériser aux alentours de 120°C, et ne se dégrade pas aux températures de soudure.

Afin de faciliter la bonne adhérence, la paroi de fixation **39** est par exemple abrasée de sorte à présenter des stries (non représentée sur les figures).

Avantageusement, la piste **35** s'étend en formant un motif, qui définit un contour **40** fictif fermé (représenté en particulier en pointillé sur la figure 7). Un tel contour **40** comprend des portions **41** latérales ainsi qu'une portion **42** de contact. Les portions latérales sont toutes les deux sensiblement rectilignes. Le contour **40** comprend en outre une portion arquée **43** décrivant un arc-de-cercle. Un tel arc de cercle permet de couvrir les sections de tubes **3, 4** à connecter entre eux, avant et après retournement. De cette manière, la zone de chauffe **44** de la plaque est optimisée. Un tel dispositif **14** de chauffe ne présente pas de deuxièmes rives **31** chaudes, lorsque la lame **11** est porté à température de soudure, évitant une chauffe incontrôlée du support **15.**

Avantageusement, le contour **40** est symétrique passant par un axe fictif passant par le milieu du dispositif **14** de chauffe. De cette façon, la chaleur se dégage de manière homogène, ce qui permet la réalisation d'une soudure de qualité.

Selon des mises en oeuvres avantageuses non représentées, le circuit électrique **34** présente plusieurs pistes **35** parallèles l'une de l'autre, toutes les pistes **35** étant reliées aux cosses **37.** De cette façon, le dégagement de chaleur est accru et optimisé.

Avantageusement, le circuit électrique **34** comprend une zone de surchauffe **45** à proximité de la pliure **21** de la feuille métallique **13,** et une zone de chauffe **44** à distance de la pliure. Une telle zone de surchauffe **45** permet d'une part, de faciliter le sectionnement du tube **3, 4,** en raison de sa proximité avec la zone de pliure **21** de la lame métallique **13,** et plus précisément de la partie tranchante **23** de ladite zone, et d'autre part, de davantage homogénéiser la température sur l'intégralité de la lame **11.**

La piste **35** comprend ainsi une fraction s'étendant au sein de la zone de surchauffe **45,** et une autre fraction s'étendant au sein de la zone de chauffe **44.**

Au sein de la zone de surchauffe **45,** la piste **35** présente une résistivité surfacique supérieure à la piste **35** s'étendant au sein de la zone de chauffe **44.** Une telle disposition est obtenue par exemple en conférant à la piste **35** parcourant la zone de surchauffe **45** une ondulation. Une telle ondulation est préférentiellement combinée à une diminution locale de la largeur de section de la piste **35.**

Avantageusement, le dispositif **14** de chauffe présente une forme complémentaire à la feuille métallique **13,** permettant d'éviter de dépasser hors de la feuille **13,** ce qui contribue à limiter l'encombrement général de la lame **11.**

Avantageusement, le dispositif **14** de chauffe présente des premières oreilles **46** faisant saillie de la première rive **30,** ce qui offre des points d'ancrage pour permettre la fixation du dispositif **14** de chauffe au support **15,** comme il peut être constaté sur la figure 5 (la feuille **13** est volontairement représentée transparente). Avantageusement, le dispositif **14** de chauffe présente des deuxièmes oreilles **47** faisant saillie de la deuxième rive **31,** ce qui offre des points d'ancrage pour permettre la mise en position du dispositif **14** de chauffe au sein de chevilles **48** reliant les faces **17** internes.

Avantageusement, le substrat **33** est réalisé dans un matériau à base d'un polymère tel qu'un polyimide. Un tel matériau offre une bonne résistance thermique et présente des caractéristiques mécaniques suffisantes pour pouvoir résister à l'effort engendré par le sectionnement.

L'on décrit à présent un dispositif de mesure de température, en l'occurrence un capteur **16** de température.

Dans les modes de réalisation représentés, le capteur **16** de température est un thermocouple s'étendant par exemple sous la forme d'une tige sensiblement plane de sorte à pouvoir être positionné dans l'entaille **32.**

Avantageusement, le capteur **16** de température présente une épaisseur inférieure à l'épaisseur de la plaque **29,** et n'impacte pas l'encombrement général de la lame **11.**

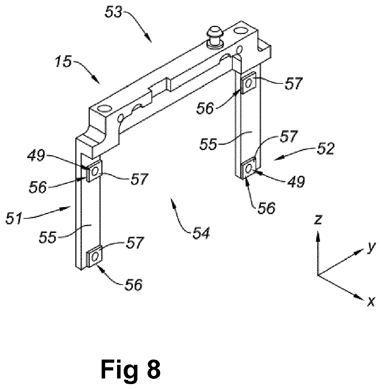

L'on se réfère désormais à la figure 8 représentant un support **15** destiné à permettre la solidarisation de la feuille **13** du dispositif **14** de chauffe et du capteur **16** de température.

Afin de maintenir la feuille pliée, le support **15** comprend avantageusement des trous **49** faisant face à des orifices **28** prévus dans la feuille, un moyen **50** de fixation tel qu'un rivet ou une vis exerce une action de pression traversant chacun des trous **49** et des orifices **28,** comme il peut être observé par exemple sur les figures 4 et 5.

Avantageusement, le support **15** comprend un premier bras **51,** un deuxième bras **52** et un corps **53** de liaison entre le premier bras **51** et le deuxième bras **52.** Le premier bras **51** et le deuxième bras **52** s'étendent dans le même sens à partir du corps **53** de liaison, conférant au support **15** une forme générale en U. De la sorte, il est formé une ouverture **54** permettant le positionnement de la feuille **13,** du dispositif **14** de chauffe et du capteur **16** de température.

Dans un mode de réalisation non représenté, les bras définissent une surface **55** de base plane, servant de surface d'appui pour les faces **24, 25** externes.

Dans le mode de réalisation représenté, la surface **55** de base présente des bossages **56** faisant saillies de la surface **55** de base. Les bossages **56** comprennent chacun un plan d'appui **57.** Les plans d'appui **57** sont avantageusement coplanaires selon une surface transversale, de sorte à former une surface d'appui, décalée par rapport à la surface **55** de base. Ainsi, les contacts entre la feuille **13** et les bras **51, 52** sont minimisés, ce qui limite la conduction thermique entre la feuille **13** et le support **15.** En outre, une telle caractéristique permet à la feuille **13,** lorsqu'elle est montée sur le support **15,** de présenter une déformation minimale. De la sorte, la feuille **13** présente un aspect plan, ce qui permet un sectionnement le plus rectiligne possible des tubes **3, 4,** et facilite l'étape de soudure subséquente. La qualité de la soudure est ainsi maîtrisée.

De manière à permettre la gestion des informations sur l'utilisation de la lame **11,** telles que le nombre d'utilisation de la lame **11,** ou la date de la dernière opération de connexion des tubes **3, 4,** la lame **11** comprend par exemple une carte **58** électronique de contrôle disposée sur le corps **53** de liaison.

La lame **11** telle que décrite ci-dessus offre de nombreux avantages, notamment :
- une utilisation possible pour la réalisation d'opérations de connexion de tubes **3, 4** pleins sans nécessiter l'utilisation de moyens supplémentaires de réduction de la poche de liquide formée au sein de la partie centrale des tubes **3, 4,** grâce à la minimisation de l'épaisseur de la lame **11,**
- une utilisation durable et pérenne, en raison de la structure de la lame **11** ;
- la possibilité de réaliser des soudures de qualité, notamment grâce à des faces **24, 25** externes non adhérentes, qui demeurent propres au gré des utilisations,
- une sécurité de manipulation par l'utilisateur, le contact entre le support et la feuille et le dispositif **14** de chauffe étant réduit,
- la possibilité de mettre en place sur le support **15,** par exemple au niveau du corps **53** de liaison, une mémoire sur un circuit électrique, grâce à une conduction thermique limitée avec la feuille **13** et le dispositif **14** de chauffe.

## Revendications

1. Lame (11) chauffante multi-usage apte à réaliser plusieurs opérations de thermo-soudure d'un tube (3, 4), comprenant une feuille métallique (13) et un dispositif (14) de chauffe étant résistif qui est apte à augmenter la température de la feuille (13) dans une plage de température prédéterminée, dans laquelle :
• la feuille métallique (13) est pliée en deux de sorte à définir un espace interne entre deux faces internes (19, 20), et comprend une face (24, 25) externe destinée à entrer en contact avec un tube (3, 4), la face externe (24, 25) étant munie d'un revêtement (26) anti-adhérent,
• le dispositif de chauffe (14) se présente sous la forme d'une plaque disposée dans l'espace interne défini par la feuille métallique (13) pliée, et est pris en sandwich entre les deux faces internes (19, 20) de ladite feuille métallique pliée (13) ;
**caractérisée en ce que** le dispositif (14) de chauffe est solidarisé par collage aux deux faces internes (19, 20) de la feuille métallique (13) au moyen d'un adhésif constitué d'une colle époxy mono-composant.

2. Lame (11) chauffante selon la revendication précédente, **caractérisée en ce que** le revêtement (26) présente plusieurs couches (27) superposées les unes sur les autres.

3. Lame (11) chauffante selon la revendication 1 ou la revendication 2, **caractérisée en ce que** le revêtement (26) comprend l'un ou plusieurs des matériaux suivants :
- PTFE (polytétrafluoroéthylène),
- PFA perfluoroalkoxy)
- FEP (fluorinated Ethylene Propylene).

4. Lame (11) chauffante selon l'une des revendications 1 à 3, **caractérisée en ce qu'**elle comprend un support (15) solidaire de la feuille (13) et du dispositif (14) de chauffe, le support (15) comprenant un corps (53) de liaison, un premier bras (51) et un deuxième bras (52) s'étendant dans le même sens de sorte à former un U, les bras (51, 52) intégrant une surface d'appui, la lame (11) étant munie de moyens (50) de fixation destinée à plaquer la feuille (13) contre la surface d'appui.

5. Lame (11) chauffante selon la revendication précédente, **caractérisée en ce que** les bras (51, 52) comprennent une surface (55) de base, les bras (51, 52) étant munis de bossages (56) faisant saillie de la surface (55) de base, définissant chacun un plan (57) d'appui décalé de la surface (55) de base, les plans (57) d'appui étant coplanaires et formant ensemble la surface d'appui.

6. Lame (11) chauffante selon l'une quelconque des revendications précédentes, **caractérisée en ce que** le dispositif (14) de chauffe comprend un substrat (33) muni d'un circuit électrique (34) comprenant une piste métallique (35).

7. Lame (11) chauffante selon la revendication précédente, **caractérisée en ce que** le substrat (33) est réalisé en un matériau appartenant à la famille des polymères polyimides.

8. Lame (11) chauffante selon la revendication 6 ou 7, **caractérisée en ce que** la piste métallique (35) s'étend en formant un motif présentant un contour (40) fictif, fermé comprenant des portions latérales (41), une portion de contact (42), toutes les deux sensiblement rectilignes, ainsi qu'une portion arquée (43) décrivant un arc-de-cercle, les portions latérales (41) étant chacune reliées d'une part à la portion arquée (43), et d'autre part à la portion de contact (42).

9. Lame (11) chauffante selon l'une quelconque des revendications 6 à 8, **caractérisée**
• **en ce que** la feuille métallique (13) est pliée en définissant une pliure (21),
• **en ce que** le circuit électrique (34) comprend une zone de surchauffe (45) au voisinage de la pliure (21) de ladite feuille métallique (13), et une zone de chauffe (44) à distance de la pliure (21),
la fraction de piste (35) s'étendant au sein de la zone de surchauffe (45) présentant une résistivité surfacique supérieure à la fraction de piste (35) s'étendant au sein de la zone de chauffe (44).

10. Lame (11) chauffante selon l'une quelconque des revendications précédentes, **caractérisée en ce qu'**elle comprend un dispositif de mesure de température disposé dans une entaille (32) pratiquée au sein du dispositif (14) de chauffe, le dispositif de mesure de température présentant une épaisseur inférieure à l'épaisseur du dispositif (14) de chauffe.

11. Lame (11) chauffante selon l'une quelconque des revendications précédentes, **caractérisée en ce que** la feuille métallique (13) présente une épaisseur de 0.15 à 0.25 millimètre, et le dispositif (14) de chauffe présente une épaisseur de 0.18 à 0.22 millimètre, la lame (11) chauffante présentant une épaisseur de 0.7 à 0.8 millimètre.

12. Machine (1) comprenant une lame (11) chauffante selon l'une quelconque des revendications précédentes.

## Patentansprüche

1. Heizelement (11) zum Mehrfachgebrauch, das dazu befähigt ist, mehrere Vorgänge zum Thermoschweißen eines Rohrs (3, 4) durchzuführen, wobei es ein Metallblatt (13) und ein Heizvorrichtung (14) umfasst, welche widerstandsbetrieben ist und dazu befähigt ist, die Temperatur des Blatts (13) innerhalb einer vorbestimmten Temperaturspannbreite zu erhöhen, wobei:
• das Metallblatt (13) derart auf sich selbst umgebogen ist, dass die beiden Innenflächen (19, 20) einen dazwischenliegenden Innenraum begrenzen, und es eine Außenfläche (24, 25) umfasst, die dazu bestimmt ist, mit einem Rohr (3, 4) in Kontakt zu treten, wobei die Außenfläche (24, 25) mit einer Antihaftbeschichtung (26) versehen ist,
• die Heizvorrichtung (14) in Form einer Platte vorliegt, die in dem Innenraum angeordnet ist, welchen das umgebogene Metallblatt (13) begrenzt, wobei sie weiterhin sich sandwichartig zwischen den beiden Innenflächen (19, 20) des umgebogenen Metallblatts (13) befindet;
**dadurch gekennzeichnet, dass** die Heizvorrichtung (14) durch Verkleben mittels eines Klebstoffs, welcher aus einem Einkomponenten-Epoxykleber besteht, fest mit den beiden Innenflächen (19, 20) des Metallblatts (13) verbunden ist.

2. Heizelement (11) nach dem vorhergehenden Anspruch, **dadurch gekennzeichnet, dass** die Beschichtung (26) mehrere Lagen (27) aufweist, die übereinander angeordnet sind.

3. Heizelement (11) nach Anspruch 1 oder Anspruch 2, **dadurch gekennzeichnet, dass** die Beschichtung (26) eines oder mehrere der folgenden Materialien umfasst:
- PTFE (Polytetrafluorethylen),
- PFA (Perfluoralkoxy)
- FEP (fluoriertes Ethylen-Propylen).

4. Heizelement (11) nach einem der Ansprüche 1 bis 3, **dadurch gekennzeichnet, dass** es einen Träger (15) umfasst, der fest mit dem Blatt (13) und der Heizvorrichtung (14) verbunden ist, wobei der Träger (15) einen Verbindungskörper (53), einen ersten Arm (51) und einen zweiten Arm (52) umfasst, welche sich derart in dieselbe Richtung erstrecken, dass sie ein U bilden, wobei die Arme (51, 52) Bestandteil einer Auflagefläche sind, wobei das Element (11) über Befestigungsmittel (50) verfügt, die dazu bestimmt sind, das Blatt (13) gegen die Auflagefläche zu drücken.

5. Heizelement (11) nach dem vorhergehenden Anspruch, **dadurch gekennzeichnet, dass** die Arme (51, 52) eine Grundfläche (55) umfassen, wobei die Arme (51, 52) mit Erhebungen (56) versehen sind, die aus Grundfläche (55) hervorstehen, sodass jeweils eine Auflageebene (57) begrenzt wird, die gegenüber der Grundfläche (55) versetzt ist, wobei die Auflageebenen (57) komplanar sind und gemeinsam die Auflagefläche bilden.

6. Heizelement (11) nach einem beliebigen der vorhergehenden Ansprüche, **dadurch gekennzeichnet, dass** die Heizvorrichtung (14) ein Substrat (33) umfasst, welches mit einem Stromkreis (34) versehen ist, der einen metallischen Leiter (35) umfasst.

7. Heizelement (11) nach dem vorhergehenden Anspruch, **dadurch gekennzeichnet, dass** das Substrat (33) aus einem Material hergestellt ist, das der Familie der Polyimidpolymere angehört.

8. Heizelement (11) nach Anspruch 6 oder 7, **dadurch gekennzeichnet, dass** der metallische Leiter (35) sich derart erstreckt, dass er ein Motiv bildet, welches eine fiktive Kontur beschreibt, die in sich geschlossen ist und seitliche Abschnitte (41), einen Kontaktabschnitt (42), wobei diese beiden im wesentlichen geradlinig verlaufen, sowie einen gebogenen Abschnitt (43) umfasst, welche einen Kreisbogen beschreibt, wobei die seitlichen Abschnitte (41) jeweils einerseits mit dem gebogenen Abschnitt (43) und andererseits mit dem Kontaktabschnitt (42) verbunden sind.

9. Heizelement (11) nach einem beliebigen der Ansprüche 6 bis 8, **dadurch gekennzeichnet**
• **dass** das Metallblatt (13) derart gebogen ist, dass eine Faltkante (21) festgelegt wird,
• **dass** der Stromkreis (34) einen Überhitzungsbereich (45) in der Nähe der Faltkante (21) des Metallblatts (13) und einen Heizbereich (44) umfasst, welcher von der Faltkante (21) beabstandet ist,
wobei der Teilbereich des Leiters (35), der sich innerhalb des Überhitzungsbereichs (45) erstreckt, einen höheren flächenbezogenen spezifischen Widerstand als der Teilbereich des Leiters (35) aufweist, der sich innerhalb des Heizbereichs (44) erstreckt.

10. Heizelement (11) nach einem beliebigen der vorhergehenden Ansprüche, **dadurch gekennzeichnet, dass** es eine Temperaturmessvorrichtung umfasst, die in einer Einkerbung (32) angeordnet ist, mit welcher die Heizvorrichtung (14) versehen wurde, wobei die Temperaturmessvorrichtung eine Dicke aufweist, welche geringer als die Dicke der Heizvorrichtung (14) ist.

11. Heizvorrichtung (11) nach einem beliebigen der vorhergehenden Ansprüche, **dadurch gekennzeichnet, dass** das Metallblatt (13) eine Dicke von 0,15 bis 0,25 Millimeter aufweist und die Heizvorrichtung (14) eine Dicke von 0,18 bis 0,22 Millimeter aufweist, wobei das Heizelement (11) eine Dicke von 0,7 bis 0,8 Millimeter aufweist.

12. Maschine (1), die ein Heizelement (11) nach einem beliebigen der vorhergehenden Ansprüche umfasst.

## Claims

1. A multi-use heating blade (11) able of carrying out several operations of heat sealing a tube (3, 4), comprising a metal sheet (13) and a heating device (14) being resistive which is able of increasing the temperature of the sheet (13) within a predetermined temperature range, wherein:
• the metal sheet (13) is folded in two so as to define an internal space between two internal faces (19, 20), and comprises an external face (24, 25) intended to come into contact with a tube (3, 4), the external face (24, 25) being provided with a non-stick coating (26),
• the heating device (14) is in the form of a plate arranged in the internal space defined by the folded metal sheet (13), and is sandwiched between the two internal faces (19, 20) of said folded metal sheet (13);
**characterised in that** the heating device (14) is secured by gluing to the two internal faces (19, 20) of the metal sheet (13) by means of an adhesive consisting of a single-component epoxy glue.

2. The heating blade (11) according to the preceding claim, **characterised in that** the coating (26) has several layers (27) superimposed on one another.

3. The heating blade (11) according to claim 1 or claim 2, **characterised in that** the coating (26) comprises one or more of the following materials:
- PTFE (polytetrafluoroethylene),
- PFA tperfluoroalkoxy)
- FEP (fluorinated ethylene propylene).

4. The heating blade (11) according to any one of claims 1 to 3, **characterised in that** it comprises a support (15) integral with the sheet (13) and the heating device (14), the support (15) comprising a connecting body (53), a first arm (51) and a second arm (52) extending in the same direction so as to form a U, the arms (51, 52) incorporating a bearing surface, the blade (11) being provided with fixing means (50) intended to press the sheet (13) against the bearing surface.

5. The heating blade (11) according to the preceding claim, **characterised in that** the arms (51, 52) comprise a base surface (55), the arms (51, 52) being provided with bosses (56) projecting from the base surface (55), each defining a bearing plane (57) offset from the base surface (55), the bearing planes (57) being coplanar and together forming the bearing surface.

6. The heating blade (11) according to any one of the preceding claims, **characterised in that** the heating device (14) comprises a substrate (33) provided with an electrical circuit (34) comprising a metal track (35).

7. The heating blade (11) according to the preceding claim, **characterised in that** the substrate (33) is made of a material belonging to the family of polyimide polymers.

8. The heating blade (11) according to claim 6 or 7, **characterised in that** the metal track (35) extends in a pattern having a fictitious, closed contour (40) comprising side portions (41), a contact portion (42), both substantially rectilinear, as well as an arcuate portion (43) describing an arc of a circle, the side portions (41) each being connected on the one hand to the arcuate portion (43), and on the other hand to the contact portion (42).

9. The heating blade (11) according to any one any of claims 6 to 8, **characterised in that**
• the metal sheet (13) is folded by defining a fold (21),
• the electrical circuit (34) comprises an overheating zone (45) in the vicinity of the fold (21) of said metal sheet (13), and a heating zone (44) remotely from the fold (21),
the track fraction (35) extending within the superheating zone (45) having a surface resistivity greater than the track fraction (35) extending within the heating zone (44).

10. The heating blade (11) according to any one of the preceding claims, **characterised in that** it comprises a temperature measuring device arranged in a cutout (32) made within the heating device (14), the temperature measuring device having a thickness less than the thickness of the heating device (14).

11. The heating blade (11) according to any one of the preceding claims, **characterised in that** the metal sheet (13) has a thickness of 0.15 to 0.25 millimetre, and the heating device (14) has a thickness of 0.18 to 0.22 millimetre, the heating blade (11) having a thickness of 0.7 to 0.8 millimetre.

12. A machine (1) comprising a heating blade (11) according to any one of the preceding claims.
